(19) Europäisches Patentamt
European Patent Office
Office européen des brevets

(11) Veröffentlichungsnummer: **0 532 962 A2**

# (12) EUROPÄISCHE PATENTANMELDUNG

(21) Anmeldenummer: **92114803.7**

(51) Int. Cl.$^5$: **C07C 65/34**, C07C 51/373

(22) Anmeldetag: **29.08.92**

(30) Priorität: **16.09.91 DE 4130725**

(43) Veröffentlichungstag der Anmeldung:
**24.03.93 Patentblatt 93/12**

(84) Benannte Vertragsstaaten:
**CH DE GB LI**

(71) Anmelder: **BASF Aktiengesellschaft**
**Carl-Bosch-Strasse 38**
**W-6700 Ludwigshafen(DE)**

(72) Erfinder: **Pfister, Juergen, Dr.**
**Ziegelofenweg 1**
**W-6720 Speyer(DE)**
Erfinder: **Schiessl, Michael**
**Rupprechtstrasse 13**
**W-6701 Altrip(DE)**
Erfinder: **Kemper, Reinhard, Dr.**
**Viernheimer Weg 41**
**W-6900 Heidelberg(DE)**

## (54) Verfahren zur Herstellung von 2-Benzoylbenzoesäuren.

(57) Verfahren zur Herstellung von 2-Benzoylbenzoesäuren durch Umsetzung von Phthalsäureanhydrid mit gegebenenfalls ein- oder mehrfach halogeniertem Benzol in Gegenwart einer Lewis-Säure als Katalysator und gegebenenfalls eines Verdünnungsmittels, wobei man die Umsetzung in einem selbstreinigenden Apparat mit Mischwirkung durchführt.

EP 0 532 962 A2

Die Vorliegende Erfindung betrifft ein neues Verfahren zur Herstellung von 2-Benzoylbenzoesäuren durch Umsetzung von Phthalsäureanhydrid mit Benzolderivaten in Gegenwart einer Lewis-Säure als Katalysator und gegebenenfalls eines Verdünnungsmittels.

Aus der BE-A-768 727 ist bekannt, Phthalsäureanhydrid mit Benzol oder dessen Derivaten in Gegenwart von Aluminiumchlorid zu 2-Benzoylbenzoesäuren umzusetzen. Problematisch bei dieser Art der Umsetzung ist, daß die Reaktionsmischung sich während der Umsetzung verfestigt und somit nicht mehr rührbar bleibt. Da die Aufarbeitung eines verfestigten Reaktionsgemisches im technischen Maßstab große Probleme mit sich bringt, muß in Gegenwart von inerten aprotischen Lösungsmitteln gearbeitet werden. Als Lösungsmittel dient häufig das Benzolderivat selbst, das dazu in großem Überschuß zur Anwendung gelangt. Nachteilig bei dieser Art der Umsetzung ist allerdings die aufwendige Isolierung der Benzoylbenzoesäure, die dadurch bedingt ist, daß große Lösungsmittelmengen abgetrennt und zurückgewonnen werden müssen. Häufig besteht auch die Gefahr einer Abwasserbelastung durch das Lösungsmittel.

Aufgabe der vorliegenden Erfindung war es nun, ein neues Verfahren zur Herstellung von 2-Benzoylbenzoesäuren bereitzustellen, das ebenfalls von Phthalsäureanhydrid und von Benzolderivaten ausgeht, bei dem aber die oben erwähnten Schwierigkeiten vermieden werden und das außerdem die Zielprodukte in guter Ausbeute und hoher Reinheit liefert. Weiterhin sollte bei der Reaktion ein Lewis-Säure-Komplex zugänglich sein, der kein Lösungsmittel und kein Benzolderivat mehr enthält, damit nach seiner Hydrolyse die direkte Isolierung der Benzoylbenzoesäuren ermöglicht und gleichzeitig eine lösungsmittelbedingte Abwasserbelastung vermieden wird.

Es wurde nun gefunden, daß die Herstellung von 2-Benzoylbenzoesäuren der Formel I

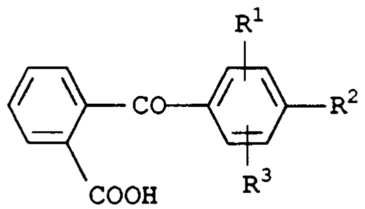 (I),

in der $R^1$, $R^2$ und $R^3$ gleich oder verschieden sind und unabhängig voneinander jeweils Wasserstoff oder Halogen bedeuten, durch Umsetzung von Phthalsäureanhydrid mit Benzolderivaten der Formel II

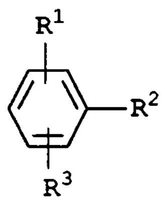 (II),

in der $R^1$, $R^2$ und $R^3$ jeweils die obengenannte Bedeutung besitzen, in Gegenwart einer Lewis-Säure als Katalysator und gegebenenfalls eines Verdünnungsmittels vorteilhaft gelingt, wenn man die Umsetzung in einem selbstreinigendem Apparat mit Mischwirkung durchführt.

Reste $R^1$, $R^2$ und $R^3$ sind z.B. Fluor, Chlor oder Brom.

Bevorzugt ist eine Verfahrensweise bei der man Phthalsäureanhydrid mit Benzolderivaten der Formel II, in der $R^1$ und $R^2$ unabhängig voneinander jeweils Wasserstoff oder Halogen und $R^3$ Wasserstoff bedeuten, umsetzt.

Besonders bevorzugt ist eine Verfahrensweise bei der man Phthalsäureanhydrid mit Halogenbenzol, insbesondere Chlorbenzol umsetzt.

Das erfindungsgemäße Verfahren wird in Gegenwart einer Lewis-Säure durchgeführt. Für das neue Verfahren geeignete Lewis-Säuren sind z.B. Aluminiumchlorid oder Aluminiumbromid.

Die Verwendung von Aluminiumchlorid als Lewis-Säure ist besonders hervorzuheben.

Das erfindungsgemäße Verfahren kann sowohl in Anwesenheit als auch in Abwesenheit eines Verdünnungsmittels durchgeführt werden, wobei die Durchführung des Verfahrens in Gegenwart eines Verdünnungsmittels bevorzugt ist.

Geeignete Verdünnungsmittel sind z.B. Salze, insbesondere Alkali- oder Erdalkalihalogenide oder Ammoniumhalogenide, wie Lithiumchlorid, Lithiumbromid, Natriumchlorid, Natriumbromid, Kaliumchlorid, Kaliumbromid, Magnesiumchlorid, Magnesiumbromid, Calciumchlorid, Calciumbromid, Ammoniumchlorid, Ammoniumbromid oder deren Mischungen.

Bevorzugt ist dabei die Verwendung von Natriumchlorid, Kaliumchlorid oder deren Mischungen.

Besonders bevorzugt ist die Verwendung von Natriumchlorid.

Im allgemeinen verwendet man je mol Lewis-Säure 0,25 bis 0,80 mol des Verdünnungsmittels.

Günstige Ergebnisse erhält man bei der gleichzeitigen Verwendung von Aluminiumchlorid als Lewis-Säure und Natriumchlorid als Verdünnungsmittel. Es hat sich als vorteilhaft erwiesen, diese Komponenten dabei im Molverhältnis Aluminiumchlorid : Natriumchlorid von 1,3 : 1 bis 4 : 1, vorzugsweise 1,7 : 1 bis 2,2 : 1 anzuwenden.

Üblicherweise kommen Phthalsäureanhydrid und Benzolderivat II in stöchiometrischen Mengen, d.h. im Molverhältnis 1 : 1, zur Anwendung. Da in der Regel aber Verluste durch verdampfendes Benzolderivat II ausgeglichen werden müssen, ist es zweckmäßig, bei einem Molverhältnis Phthalsäureanhydrid : Benzolderivat II von 1 : 1 bis 1 : 3, vorzugsweise 1 : 1,2 bis 1 : 2 zu arbeiten.

Je mol Phthalsäureanhydrid kommen im allgemeinen 2 bis 10 mol, vorzugsweise 2 bis 3 mol, Lewis-Säure zur Anwendung.

Das erfindungsgemäße Verfahren wird in einem selbstreinigenden Apparat mit Mischwirkung durchgeführt, der vorteilhaft über ein hohes Reaktionsvolumen verfügt. Solche Apparate sind an sich bekannt und handelsüblich. Geeignete Reaktoren dieser Art sind z.B. Misch- und Knetreaktoren oder Schnekkenreaktoren, wobei die Durchführung des erfindungsgemäßen Verfahrens in Misch- und Knetreaktoren bevorzugt ist. Beispielhaft seien der Allphasenreaktor oder der Reaktor Discotherm B der Fa. List genannt.

Besonders hervorzuheben ist die Verwendung eines großvolumigen, vorzugsweise kontinuierlich arbeitenden, Knetreaktors (z.B. Allphasenreaktor, Fa. List), der sich durch eine intensive Misch- und Knetwirkung, einen hohen Selbstreinigungsgrad, eine große Wärmeaustauschfläche, ein großes Nutzvolumen und eine enge Verweilzeitverteilung auszeichnet.

Ein solcher Apparat ist beispielsweise in CZ-Chemie-Technik, Seiten 419 bis 423, 1973, beschrieben.

Das neue Verfahren kann in diskontinuierlicher Arbeitsweise oder vorzugsweise in kontinuierlicher Arbeitsweise durchgeführt werden.

Die Umsetzung von Phthalsäureanhydrid mit dem Benzolderivat II wird im allgemeinen bei Normaldruck oder leicht erniedrigtem Druck (bis zu ca. 900 mbar) und bei einer Temperatur von 20 bis 140 °C, vorzugsweise 60 bis 100 °C und insbesondere 70 bis 95 °C, vorgenommen.

Die Reaktionszeit, d.h. die Verweilzeit im selbstreinigenden Apparat mit Mischwirkung beträgt üblicherweise 5 bis 180 Minuten, vorzugsweise 5 bis 60 Minuten und insbesondere 10 bis 30 Minuten.

Das Schergefälle im selbstreinigenden Apparat mit Mischwirkung beträgt üblicherweise 50 bis 20000 $sec.^{-1}$, vorzugsweise von 100 bis 600 $sec^{-1}$. Der spezifische Energieeintrag durch die Scherung beträgt in der Regel 0,01 bis 0,3 kWh/kg.

Das erfindungsgemäße Verfahren wird zweckmäßig so durchgeführt, daß die Zugabe von Phthalsäureanhyrid, Benzolderivat II, Lewis-Säure und gegebenenfalls Verdünnungsmittel in den Reaktor als Gemisch oder getrennt voneinander erfolgt. Bevorzugt ist eine Variante, bei der die Lewis-Säure und gegebenenfalls das Verdünnungsmittel gemeinsam mit Phthalsäureanhyrid und dem Benzolderivat II in vorgemischtem Zustand in den Reaktor dosiert werden.

Unter intensiver Misch- und Knetwirkung erfolgt die Erwärmung der in den Reaktor dosierten Reaktionsteilnehmer auf die obengenannte Temperatur. Dies führt zu einer Komplexbildung aus entstehender Benzoylbenzoesäure I und Lewis-Säure. Der bei Anwendung von Aluminiumchlorid bei der Reaktion gebildete Chlorwasserstoff wird während der Umsetzung gemeinsam mit einem Teil des nicht umgesetzten Benzolderivats II über die Entgasungsdome aus dem Reaktor abgeführt. Bevor nach beendeter Reaktion der Komplex aus Zielprodukt und Lewis-Säure aus dem Reaktor ausgetragen wird, kann überschüssiges, nicht umgesetztes Benzolderivat II über die Entgasungsdome abdestilliert werden. Das auf diese Weise zurückgewonnene Benzolderivat II kann ohne weitere Reinigung in das erfindungsgemäße Verfahren zurückgeführt werden.

Zur Abtrennung des Benzolderivats II vom Komplex aus Zielprodukt und Lewis-Säure kann es vorteilhaft sein, den Reaktor unter vermindertem Druck zu betreiben.

Nach beendeter Umsetzung und Austrag aus dem Reaktor wird das Reaktionsgemisch mit wäßriger Säure behandelt. Die Benzoylbenzoesäure I fällt dabei in kristalliner Form an und kann z.B. durch Filtrieren oder Zentrifugieren von der wäßrigen Phase abgetrennt und durch Waschen mit Wasser gereinigt werden.

Die Behandlung mit wäßriger Säure kann mit Mineralsäuren, z.B. Salzsäure oder verdünnte Schwefelsäure, vorgenommen werden. Die Verwendung von Salzsäure ist bevorzugt.

Führt man das erfindungsgemäße Verfahren mit Aluminiumchlorid als Lewis-Säure durch, so ist es zweckmäßig, den während der Umsetzung freiwerdenden Chlorwasserstoff in Wasser zu lösen und die so resultierende Salzsäure für die wäßrig-saure Behandlung zu verwenden.

Die Behandlung mit wäßriger Säure kann entweder diskontinuierlich oder vorzugsweise kontinuierlich durchgeführt werden.

Das erfindungsgemäße Verfahren zeichnet sich durch eine Reihe von Vorteilen aus. So ist die Reaktionszeit, d.h. die Verweilzeit im Reaktor, sehr kurz, wodurch ein selektiver Reaktionsverlauf gewährt ist. Die Zielprodukte fallen in hoher Reinheit an. Weiterhin ist auf die günstige Raum-Zeit-Ausbeute im neuen Verfahren hinzuweisen. Zusätzlich kann ein hoher Überschuß an einem der beiden Reaktionspartner vermieden werden, d.h. die Reaktanden werden annähernd in stöchiometrischem Verhältnis zur Reaktion gebracht. Schließlich kann auf die Anwesenheit eines organischen Lösungsmittels verzichtet werden, wodurch die Isolierung des Zielprodukts stark vereinfacht und die Abwasserbelastung verringert wird.

Bei den Benzoylbenzoesäuren der Formel I handelt es sich um wertvolle Zwischenprodukte für die Herstellung von Anthrachinonderivaten und -farbstoffen.

Die folgenden Beispiele sollen die Erfindung näher erläutern.

Beispiel 1

In einem Allphasenreaktor vom Typ AP12 Conti (Fa. List) wurde durch eine Stauscheibe ein Füllvolumen von 6,8 l vorgegeben. Die Drehzahl der Putzwelle betrug 40 U/min. Der Druck im Reaktor wurde auf 990 mbar eingestellt.

In diesen Misch- und Knetreaktor wurden kontinuierlich 30,9 kg/h eines Gemisches, das aus 5,91 kg (39,93 mol) Phthalsäureanhydrid, 13,32 kg (99,92 mol) Aluminiumchlorid, 2,66 kg (45,47 mol) Natriumchlorid und 9,00 kg (80,00 mol) Chlorbenzol bestand, zudosiert. Das Gemisch schmolz und reagierte bei einer Temperatur von 95 °C. Der bei der Reaktion entstehende Chlorwasserstoff sowie nicht abreagiertes Chlorbenzol wurden nach Austritt aus dem Reaktor über eine Kühlfalle geleitet. Das dabei auskondensierte Chlorbenzol (4,8 kg/h) wurde in die Chlorbenzolvorlage zurückgeführt und als Edukt wiederverwendet. Die Verweilzeit im Reaktor betrug 20 Minuten.

Der resultierende Aluminiumkomplex (25,99 kg/h) wurde 30 Minuten bei 90 bis 100 °C mit 75 kg 10 gew.%iger Salzsäure hydrolysiert. Die kristallin anfallende 2-(4-Chlorbenzoyl)benzoesäure wurde über eine Filterpresse abgesaugt, mit 180 kg Wasser neutral gewaschen und getrocknet. Ausbeute 9,75 kg (95 % d. Th.); Reinheit > 95 %.

In analoger Weise werden die in der folgenden Tabelle aufgeführten Benzoylbenzoesäuren erhalten.

| Beispiel Nr. | Benzoylbenzoesäure | Benzolderivat |
|---|---|---|
| 2 | 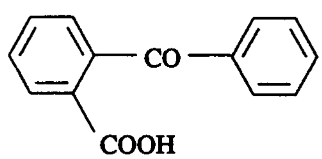 | Benzol |
| 3 | 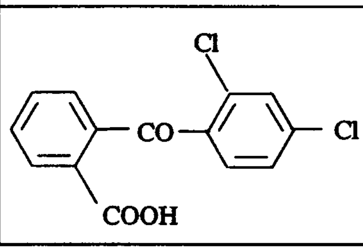 | 1,3-Dichlorbenzol |

**Patentansprüche**

**1.** Verfahren zur Herstellung von 2-Benzoylbenzoesäuren der Formel I

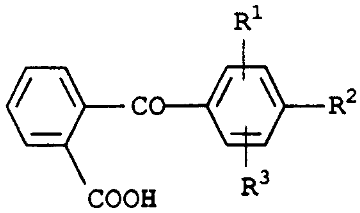

(I),

in der $R^1$, $R^2$ und $R^3$ gleich oder verschieden sind und unabhängig voneinander jeweils Wasserstoff oder Halogen bedeuten, durch Umsetzung von Phthalsäureanhydrid mit Benzolderivaten der Formel 11

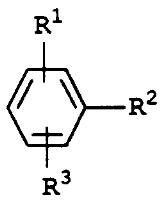

(II),

in der $R^1$, $R^2$ und $R^3$ jeweils die obengenannte Bedeutung besitzen, in Gegenwart einer Lewis-Säure als Katalysator und gegebenenfalls eines Verdünnungsmittels, dadurch gekennzeichnet, daß man die Umsetzung in einem selbstreinigendem Apparat mit Mischwirkung durchführt.

2. Verfahren nach Anspruch 1, dadurch gekennzeichnet, daß man Phthalsäureanhydrid mit Benzolderivaten der Formel II, in der $R^1$ und $R^2$ unabhängig voneinander jeweils Wasserstoff oder Halogen und $R^3$ Wasserstoff bedeuten, umsetzt.

3. Verfahren nach Anspruch 1, dadurch gekennzeichnet, daß man als Lewis-Säure Aluminiumchlorid oder Aluminiumbromid verwendet.

4. Verfahren nach Anspruch 1, dadurch gekennzeichnet, daß man die Umsetzung in Gegenwart eines Verdünnungsmittels durchführt.

5. Verfahren nach Anspruch 1, dadurch gekennzeichnet, daß man als Verdünnungsmittel Natriumchlorid, Kaliumchlorid oder deren Mischungen verwendet.